(19) Europäisches Patentamt
European Patent Office
Office européen des brevets

(11) EP 4 556 522 A1

(12) **EUROPEAN PATENT APPLICATION**
published in accordance with Art. 153(4) EPC

(43) Date of publication:
**21.05.2025 Bulletin 2025/21**

(21) Application number: **23860946.5**

(22) Date of filing: **01.09.2023**

(51) International Patent Classification (IPC):
***C08L 3/12*** *(2006.01)*

(52) Cooperative Patent Classification (CPC):
**C08B 30/20; C08B 31/00; C08B 35/02; C08J 3/24; C08L 3/02; C08L 3/12**

(86) International application number:
**PCT/KR2023/013094**

(87) International publication number:
**WO 2024/049271 (07.03.2024 Gazette 2024/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.09.2022 KR 20220110608**

(71) Applicant: **LG Chem, Ltd.**
**Yeongdeungpo-gu**
**Seoul 07336 (KR)**

(72) Inventors:
- **HAM, Kyung Rok**
  **Daejeon 34122 (KR)**
- **CHOI, Hyung Sam**
  **Daejeon 34122 (KR)**
- **YUN, Hae Sung**
  **Daejeon 34122 (KR)**
- **CHO, Beom Shin**
  **Daejeon 34122 (KR)**
- **CHUNG, Da Sol**
  **Daejeon 34122 (KR)**

(74) Representative: **Plasseraud IP**
**104 Rue de Richelieu**
**CS92104**
**75080 Paris Cedex 02 (FR)**

# (54) POLYMER COMPOSITION

(57) The present invention relates to a polymer composition and a use thereof. The present invention can provide a polymer composition having excellent biodegradability and absorption capacity, and a use thereof. The present invention can provide the polymer composition and a use thereof by controlling the ratio of amylose and amylopectin in a polysaccharide component included in the polymer composition and the molecular weight of the polysaccharide component.

[Figure 1]

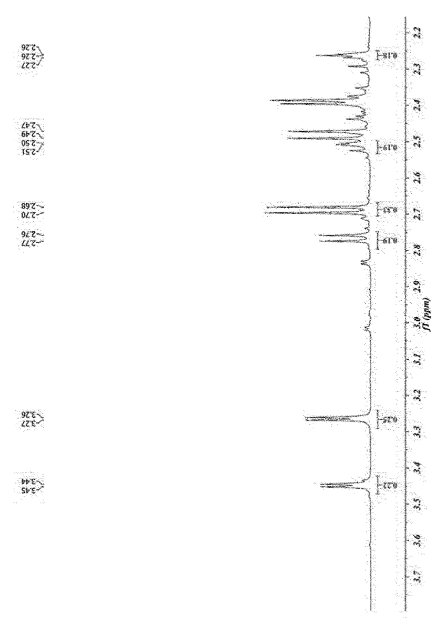

5

EP 4 556 522 A1

## Description

### Technical Field

This application claims the benefit of priority based on Korean Patent Application No. 10-2022-0110608 dated September 1, 2022, the disclosure of which is incorporated herein by reference in its entirety.
The present application relates to a polymer composition and a use thereof.

### Background Art

A hydrogel polymer or hydrogel is generally defined as a cross-linked hydrophilic polymer.
Such a polymer can be used as a material called an SAP (Super Absorbent Polymer). The SAP is a material that can absorb moisture tens to thousands of times its own weight. The SAP is used for various applications, such as sanitary products such as hygiene products or diapers, medical products, household materials, agricultural materials, horticultural materials, transportation materials, civil engineering and construction materials, materials related to electrical and electronic devices, or water treatment agents.
The most widely used hydrogel polymer, which is used as the SAP, is a polymer made of a vinyl-based material such as cross-linked polyacrylic acid.
Such materials are relatively inexpensive and have excellent water absorption capacity, but cause various problems because they remain semi-permanently even after disposal.
To solve such problems, there are various attempts to manufacture the SAP from so-called biodegradable materials.
However, the materials known to date do not form the SAP with balanced physical properties. For example, the most representative physical property required for the SAP is absorption capacity, but in the SAP of a biodegradable material known to date, at least one characteristic of absorption capacity and biodegradability is not satisfactorily secured, or in some cases, both physical properties are not secured at an appropriate level.

### Disclosure

### Technical Problem

The present application is intended to provide a polymer composition capable of securing simultaneously excellent absorption capacity and biodegradability, and a use thereof.

### Technical Solution

Among the physical properties mentioned in this specification, the value of the physical property affected by the measurement temperature is the result measured at room temperature, unless specifically stated otherwise.
In this specification, the term room temperature is a natural temperature without artificially warming and cooling, which may mean, for example, any one temperature in a range of about 10°C to 30°C, or a temperature of 23°C or 25°C or so.
Among the physical properties mentioned in this specification, the value of the physical property affected by the measurement pressure is the result measured at normal pressure, unless specifically stated otherwise.
In this specification, the term normal pressure is a pressure of general atmospheric pressure or so that is not artificially pressurized or depressurized, which may mean, for example, a pressure of about 740 mmHg to 780 mmHg or so.
Among the physical properties mentioned in this specification, the value of the physical property affected by the measured humidity is the result measured at humidity in the standard state, unless otherwise specified.
In this specification, the humidity in the standard state means a humidity of about 40%, 50%, or 60% or so as the relative humidity.
In this specification, the term alkyl or alkyl group means an alkyl or alkyl group with 1 to 20 carbon atoms, 1 to 16 carbon atoms, 1 to 12 carbon atoms, 1 to 8 carbon atoms, or 1 to 4 carbon atoms, unless otherwise specified. Such an alkyl or alkyl group may be linear, branched, or cyclic. Such an alkyl or alkyl group may also be optionally substituted with one or more substituents.
In this specification, unless otherwise specified, the term alkylene or alkylene group means a functional group which is connected to another object by separating two hydrogen atoms from an alkane, where it has a structure that the two hydrogen atoms are separated from other carbon atoms of the alkane. Such an alkylene or alkylene group may be an alkylene or alkylene group with 2 to 20 carbon atoms, 2 to 16 carbon atoms, 2 to 12 carbon atoms, 2 to 8 carbon atoms, or 2 to 4 carbon atoms. Such an alkylene or alkylene group may be linear, branched, or cyclic. Such an alkylene or alkylene

group may also be optionally substituted with one or more substituents.

In this specification, unless otherwise specified, the term alkylidene or alkylidene group means a functional group which is connected to another object by separating two hydrogen atoms from an alkane, where it has a structure that the two hydrogen atoms are separated from one carbon atom of the alkane. Such an alkylidene or alkylidene group may be an alkylidene or alkylidene group with 1 to 20 carbon atoms, 1 to 16 carbon atoms, 1 to 12 carbon atoms, 1 to 8 carbon atoms, or 1 to 4 carbon atoms. Such an alkylidene or alkylidene group may be linear, branched, or cyclic. Such an alkylidene or alkylidene group may also be optionally substituted with one or more substituents.

In this specification, the term hydrogel polymer or hydrogel polymer composition may mean an absorbent material containing a polymer, and such a material may also be simply referred to as a hydrogel.

In this specification, the term absorbent material means a material exhibiting at least one characteristic of moisture content, centrifuge retention capacity (CRC), and absorption capacity under pressure (AUP) as defined in this specification.

In this specification, the biodegradable material means a material exhibiting a degree of biodegradability as defined herein.

The present application relates to a polymer composition. The polymer composition may be simultaneously the absorbent material and the biodegradable material as described above.

In this specification, the term polymer composition means a material comprising a polymer. Here, the term composition means a material comprising two or more components. In this instance, the two or more components do not necessarily have to be different types of components. For example, a polysaccharide component to be described below may be composed of polysaccharide polymers with two molecules or more, and in this case, even when only the polysaccharide component is present, the relevant material may be called a polymer composition. Also, two or more components included in the polymer composition may perform physical or chemical interaction or reaction with each other, or may also be in a state of being simply mixed without such interaction or reaction. The polymer may mean a material formed by connecting two or more unitary bodies by covalent bonds. In one example, the polymer may be a material including a structure in which two or more unitary bodies are connected by covalent bonds and having a molecular weight of more than or equal to a certain level. The range of the molecular weight is not limited, but the molecular weight of the polymer may be approximately 500 g/mol or more in terms of weight average molecular weight (Mw). The upper limit of the weight average molecular weight is not particularly limited, and for example, the weight average molecular weight of the polymer may be about 1,000,000 g/mol or less or so.

The weight average molecular weight is a value measured by a method described in "4. Molecular weight measurement of polysaccharide component" of Example sections in this specification.

In one example, the lower limit of the polymer content in the polymer composition may be about 55 wt%, 60 wt%, 65 wt%, 70 wt%, 75 wt%, 80 wt%, 85 wt%, 90 wt%, or 95 wt% or so, and the upper limit thereof may be 100 wt%, 100 wt%, 98 wt%, 96 wt%, 94 wt%, 92 wt%, or 90 wt% or so. The content may also be within a range of more than or equal to, or more than any one of the above-described lower limits; or within a range of less than or equal to, or less than any one of the above-described upper limits while being more than or equal to, or more than any one of the above-described lower limits. The content of the polymer is a content based on the solid content, and therefore, when the polymer composition comprises a solvent, the content is a ratio to the weight of the entire polymer composition excluding the solvent.

The polymer composition may comprise a polysaccharide component.

The term polysaccharide component means a component composed of one or more polysaccharides. That is, the polysaccharide component comprises only polysaccharides. The polysaccharide contained in the polysaccharide component may be one or, two or more types. Here, the category of two or more polysaccharides may also include polysaccharides with different chemical structures (e.g., types of repeating units, etc.) and/or polysaccharides with the same or substantially similar chemical structure, but different physical properties such as the molecular weight.

The term polysaccharide has a meaning known in the industry. The polysaccharide generally refers to a polymer molecule in which two or more unitary bodies are linked by covalent bonds. Here, in one example, the covalent bond connecting the unitary bodies may be a glycosidic bond. The polysaccharide is a polymer molecule, that is, a polymer, which may have a weight average molecular weight in the above-mentioned range.

The unitary body forming the polysaccharide may be a biomolecule composed of carbon, hydrogen, and oxygen, or composed of carbon, hydrogen, oxygen, and nitrogen. In this specification, the term biomolecule is interpreted to have the meaning generally applied in the industry. Typically, as an example of the biomolecule in the industry, monosaccharides such as glucose, galactose, fructose or xylose, disaccharides such as sucrose, lactose, maltose or trehalose, polyols such as sorbitol or mannitol, oligosaccharides such as maltodextrin, dextrin, raffinose, stachyose or fructo-oligosaccharide and/or amino sugars such as glucosamine or N-acetal glucosamine, and the like are known, but the types of biomolecules in the present application are not limited to the foregoing.

In one example, the polymer composition may also be in a powder or particle state formed through a grinding process or the like.

The polymer composition may comprise only the polysaccharide component, or may further comprise other

components in addition to the polysaccharide component, as the polymer. In the polymer composition, the polysaccharide component may exist in a cross-linked state.

Examples of other components that may be included in the polymer composition together with the polysaccharide are not particularly limited, but may be exemplified by a cross-linking agent cross-linking the polysaccharide, or a polymer different from the polysaccharide, and the like.

In one example, the lower limit of the weight ratio of the polysaccharide component in the polymer composition may be 55 wt%, 60 wt%, 65 wt%, 70 wt%, 75 wt%, 80 wt%, 85 wt%, 90 wt%, 92 wt%, 94 wt%, 96 wt%, or 98 wt% or so, and the upper limit thereof may be 100 wt%, 98 wt%, 96 wt%, 94 wt%, 92 wt%, or 90 wt% or so. The weight ratio may also be within a range of more than or equal to, or more than any one of the above-described lower limits; or within a range of less than or equal to, or less than any one of the above-described upper limits while being more than or equal to, or more than any one of the above-described lower limits. The weight ratio is a ratio based on the solid content, and therefore, when the polymer composition comprises a solvent, the ratio is a ratio to the weight of the entire polymer composition excluding the solvent.

The higher the ratio of the polysaccharide component in the polymer composition, the greater the biodegradability of the polymer composition. By applying a specific type of polysaccharide component as the polysaccharide component, appropriate cross-linking can be performed, and the cross-linked material can exhibit excellent absorption capacity and biodegradability at the same time.

When the polymer composition is an absorbent material, the lower limit of the moisture content of the polymer composition may be 40 wt%, 45 wt%, 50 wt%, or 55 wt% or so, and the upper limit thereof may be 70 wt%, 65 wt%, or 60 wt% or so. The moisture content may also be within a range of more than or equal to, or more than any one of the above-described lower limits; or within a range of less than or equal to, or less than any one of the above-described upper limits while being more than or equal to, or more than any one of the above-described lower limits.

The moisture content is the content ratio of moisture contained in the polymer composition relative to the total weight of the polymer composition to be measured, which may be calculated through the weight of the polymer composition containing moisture and the weight of the dried polymer composition. For example, the moisture content may be calculated through the weight loss due to evaporation of moisture in the polymer composition during a process of drying the polymer composition in a crumb state through infrared heating. The drying process for measuring the moisture content may comprise raising the temperature from room temperature to about 50°C or so and then performing vacuum drying for about 6 hours or so while maintaining 50°C. The polymer composition may exhibit the water content before or after cross-linking.

When the polymer composition is an absorbent material, the lower limit of the centrifuge retention capacity (CRC) of the polymer composition according to EDANA (European Disposables and Nonwovens Association) method WSP 241.3 may be 12 g/g, 13 g/g, 14 g/ g, 15 g/g, 20 g/g, 25 g/g, 30 g/g, 35 g/g, 40 g/g, or 45 g/g or so, and the upper limit thereof may be 60 g/g, 55 g/ g, 50 g/g, 45 g/g, 40 g/g, 35 g/g, 34 g/g, 33 g/g, or 32 g/g or so. The centrifuge retention capacity may also be within a range of more than or equal to, or more than any one of the above-described lower limits; or within a range of less than or equal to, or less than any one of the above-described upper limits while being more than or equal to, or more than any one of the above-described lower limits. The centrifuge retention capacity is evaluated by a method described in "1. Evaluation of centrifuge retention capacity (CRC)" of Example sections in this specification. The polymer composition may exhibit the centrifuge retention capacity before or after cross-linking.

When the polymer composition is an absorbent material, the lower limit of absorption capacity under pressure (AUP) of the polymer composition at 0.7 psi according to EDANA (European Disposables and Nonwovens Association) method WSP 242.3 may be 1.5 g/g, 2 g/g, 2.5 g/g, 3 g/g, 3.5 g/g, 4 g/g, 4.5 g/g, 5 g/g, or 5.5 g/g or so, and the upper limit thereof may be 40 g/g, 35 g/g, 30 g/g, 20 g/g, 15 g/g, 10 g/g, 8 g/g, 6 g/g, or 4 g/g or so. The absorption capacity under pressure may also be within a range of more than or equal to, or more than any one of the above-described lower limits; or within a range of less than or equal to, or less than any one of the above-described upper limits while being more than or equal to, or more than any one of the above-described lower limits. The absorption capacity under pressure is evaluated by a method described in "2. Evaluation of absorption capacity under pressure (AUP)" of Example sections in this specification. The polymer composition may exhibit the absorption capacity under pressure before or after cross-linking.

If the polymer composition exhibits at least one characteristic of the moisture content, centrifuge retention capacity, and absorption capacity under pressure as described above, the relevant material may be defined as an absorbent material.

The polymer composition may exhibit excellent biodegradability. For example, the polymer composition may be the absorbent material and simultaneously the biodegradable material. For example, the lower limit of the biodegradability of the polymer composition may be 60%, 62%, 64%, 66%, 68%, 70%, 72%, 74%, 76%, 78%, 80%, 82%, 84%, 86%, 88%, 90%, 92%, 94%, 96%, 98%, or 99% or so, and the upper limit thereof may be 100%, 98%, 96%, 94%, 92%, 90%, 88%, 86%, 84%, 82%, 80%, 78%, or 76% or so. The biodegradability may also be within a range of more than or equal to, or more than any one of the above-described lower limits; or within a range of less than or equal to, or less than any one of the above-described upper limits while being more than or equal to, or more than any one of the above-described lower limits.

The biodegradability is evaluated by a method described in "3. Measurement of biodegradability" of Example sections in this specification. The polymer composition may exhibit the biodegradability before or after cross-linking.

The polymer composition can exhibit the excellent absorptivity and biodegradability simultaneously.

Such a polymer composition can be implemented by applying a polysaccharide component to be described below as the polysaccharide component. In general, the polysaccharide-based polymer composition has excellent biodegradability, but has low water absorption capacity and low cross-linking efficiency for application as an absorbent material. Therefore, in general, to form an absorbent material using a polysaccharide-based material, it is obtained by blending other components with the polysaccharide without applying only the polysaccharide. However, when a specific type of polysaccharide component is applied as the polysaccharide component, only the polysaccharide component cans secure an efficient cross-linking rate to be described below, and can simultaneously exhibit excellent absorption capacity and biodegradability after cross-linking.

As described above, the polysaccharide is a polymeric substance in which two or more unitary bodies are linked by covalent bonds such as glycosidic bonds, and the representatively known polysaccharide includes starch, dextrin, or chitosan, and the like.

The polysaccharide component may comprise one or more selected from the group consisting of the starch, dextrin, and chitosan.

In one example, the polysaccharide component may comprise amylose and amylopectin.

Amylopectin and amylose are types of polysaccharides mainly found in plants, and the starch of polysaccharides is composed of amylose and amylopectin. The amylose is composed of glucose molecules linked by $\alpha$ (1$\rightarrow$4) glycosidic bonds and has a linear chain structure, whereas the amylopectin has relatively short and highly branched chains. The amylose crystallizes relatively easily compared to the amylopectin, and the amylopectin has a relatively higher solubility in water than the amylose.

In this specification, it has been confirmed that the ratio of amylose and amylopectin in the polysaccharide component and the molecular weight of the relevant polysaccharide component are significantly related to the absorption capacity and biodegradability of the polymer composition.

As the ratio of amylopectin in the polysaccharide component decreases and the ratio of amylose increases, the biodegradability of the material increases, but the absorption capacity tends to decrease somewhat. In addition, as the molecular weight of the polysaccharide component increases, the biodegradability of the material decreases, but the absorption capacity tends to increase. Therefore, by controlling the amounts of amylose and amylopectin and the molecular weight of the polysaccharide component, a material with excellent biodegradability and absorption capacity can be obtained.

Accordingly, the polymer composition may have F in a predetermined range according to Equation 1 below.

[Equation 1]

$$F = Log(Mw \times \frac{Ap}{Ap+Am})$$

In Formula 1, Ap is the ratio of the amylopectin in the polysaccharide component, Am is the ratio of the amylose in the polysaccharide component, and Mw is the weight average molecular weight of the polysaccharide component.

F in Formula 1 is a factor determined according to the relationship between the amounts of amylose and amylopectin in the polysaccharide component and the molecular weight of the polysaccharide component. For example, the value of F above increases as the molecular weight of the polysaccharide component increases and/or the ratio of amylopectin in the polysaccharide component increases.

The polysaccharide component whose amylose/amylopectin ratio and molecular weight are controlled so that F in Formula 1 is in a range to be described below may be efficiently cross-linked to form a material with excellent absorption capacity and biodegradability.

In Equation 1, Ap and Am are the ratios of amylopectin and amylose measured by a method described in "5. Content measurement of amylopectin and amylose in polysaccharide component" of Example sections in this specification, and the unit thereof is %.

In Equation 1, Mw is the weight average molecular weight of the polysaccharide component, which is a value measured by a method described in "4. Molecular weight measurement of polysaccharide component" of Examples sections in this specification, and the unit thereof is g/mol.

The lower limit of F in Equation 1 may be 4, 4.2, 4.4, 4.6, 4.8, 5.0, 5.2, 5.4, 5.6, 5.8, 6.0, 6.2, 6.4, 6.6, 6.8, 7.0, 7.2, 7.4, 7.6, 7.8, 8.0, or 8.2 or so, and the upper limit thereof may be 20, 18, 16, 14, 12, 10, 9, 8, 7, 6, or 5 or so. F in Equation 1 may also be within a range of more than or equal to, or more than any one of the above-described lower limits; or within a

range of less than or equal to, or less than any one of the above-described upper limits while being more than or equal to, or more than any one of the above-described lower limits.

Within such a range, the polysaccharide component may be efficiently cross-linked to form a polymer composition with excellent absorption capacity and biodegradability.

In Equation 1, the ranges of Mw, Am, and Ap may be adjusted to an appropriate level.

In one example, the lower limit of Mw in Equation 1 may be 200,000 g/mol, 250,000 g/mol, 300,000 g/mol, 350,000 g/mol, 400,000 g/mol, 450,000 g/mol, 500,000 g/mol, 550,000 g/mol, 600,000 g/mol, 650,000 g/mol, 700,000 g/mol, 750,000 g/mol, 800,000 g/mol, 850,000 g/mol, 900,000 g/mol, 950,000 g/mol, 1,000,000 g/mol, 1,500,000 g/mol, 2,000,000 g/mol, 2,500,000 g/mol, 3,000,000 g/mol, 3,500,000 g/mol, 4,000,000 g/mol, 4,500,000 g/mol, 5,000,000 g/mol, 5,500,000 g/mol, 6,000,000 g/mol, 6,500,000 g/mol, 7,000,000 g/mol, 7,500,000 g/mol, 8,000,000 g/mol, 8,500,000 g/mol, 9,000,000 g/mol, 9,500,000 g/mol, 10,000,000 g/mol, 20,000,000 g/mol, 30,000,000 g/mol, 40,000,000 g/mol, 50,000,000 g/mol, 60,000,000 g/mol, 70,000,000 g/mol, 80,000,000 g/mol, 90,000,000 g/mol, 100,000,000 g/mol, 110,000,000 g/mol, 120,000,000 g/mol, 130,000,000 g/mol, 140,000,000 g/mol, 150,000,000 g/mol, 160,000,000 g/mol, 170,000,000 g/mol, or 180,000,000 g/mol or so, and the upper limit thereof may be 1,000,000,000 /mol, 900,000,000 /mol, 800,000,000 /mol, 700,000,000 /mol, 600,000,000 /mol, 500,000,000 /mol, 400,000,000 /mol, 300,000,000 /mol, 200,000,000 /mol, 150,000,000 /mol, 100,000,000 /mol, 90,000,000 /mol, 80,000,000 /mol, 70,000,000 /mol, 60,000,000 /mol, 50,000,000 /mol, 40,000,000 /mol, 30,000,000 /mol, 20,000,000 /mol, 10,000,000 /mol, 9,000,000 /mol, 8,000,000 /mol, 7,000,000 /mol, 6,000,000 /mol, 5,000,000 /mol, 4,000,000 /mol, 3,000,000 /mol, 2,000,000 /mol, 1,000,000 /mol, 900,000 /mol, 800,000 /mol, 700,000 /mol, 600,000 /mol, or 500,000 /mol or so. The Mw may also be within a range of more than or equal to, or more than any one of the above-described lower limits; or within a range of less than or equal to, or less than any one of the above-described upper limits; or within a range of less than or equal to, or less than any one of the above-described upper limits while being more than or equal to, or more than any one of the above-described lower limits.

The lower limit of Ap in Equation 1 may be 5%, 10%, 15%, 20%, 25%, 30%, 35%, 40%, 45%, 50%, 55%, 60%, 65%, 70%, 75%, or 80% or so, and the upper limit thereof may be 95%, 90%, 85%, 80%, 75%, 70%, 65%, 60%, 55%, 50%, 45%, 40%, 35 %, 30%, 35%, or 20% or so. The Ap may also be within a range of more than or equal to, or more than any one of the above-described lower limits; or within a range of less than or equal to, or less than any one of the above-described upper limits; or within a range of less than or equal to, or less than any one of the above-described upper limits while being more than or equal to, or more than any one of the above-described lower limits.

The lower limit of Am in Equation 1 may be 5%, 10%, 15%, 20%, 25%, 30%, 35%, 40%, 45%, 50%, 55%, 60%, 65%, 70%, 75%, or 80% or so, and the upper limit thereof may be 95%, 90%, 85%, 80%, 75%, 70%, 65%, 60%, 55%, 50%, 45%, 40%, 35 %, 30%, 35%, or 20% or so. The Am may also be within a range of more than or equal to, or more than any one of the above-described lower limits; or within a range of less than or equal to, or less than any one of the above-described upper limits; or within a range of less than or equal to, or less than any one of the above-described upper limits while being more than or equal to, or more than any one of the above-described lower limits.

In one example, the sum of Am and Ap above in the polysaccharide component may be 100%.

In one example, when the polysaccharide of the polysaccharide component is a modified polysaccharide to be described below, the functional group introduced into the modified polysaccharide may be introduced into either amylose or amylopectin, or may be introduced into both. The reaction of introducing the functional group, which is described below, can occur in both amylose and amylopectin, but more efficient modification may be possible when the content of amylopectin is greater than the content of amylose.

As the polysaccharide component, an appropriate type may be selected and used among the above-mentioned polysaccharides if it contains at least polysaccharides including amylose and amylopectin.

One, or two or more of known polysaccharides are combined to form a polysaccharide component so that F in Formula 1 is within the above-described range, which may be applied to the polymer composition.

Among known polysaccharides, starch may typically be used. When starch is used, starch having a gelatinization temperature in a range of about 50°C to 90°C and a peak viscosity (BU) in a range of 50 to 1000 may be used.

As the starch, any known starch may be applied without special limitation, and for example, one or two or more types selected from potato starch, corn starch, rice starch, wheat starch, tapioca starch, and polymer starch may be applied.

Such a polysaccharide may comprise at least two or more monosaccharide unitary bodies linked by covalent bonds (e.g., glycosidic bonds). At this time, the monosaccharide unitary body may be exemplified by the above-described biomolecule, and may be specifically exemplified by glucose, galactose, fructose, xylose, glucosamine, or N-acetalglucosamine, and the like, but is not limited thereto.

Among the unitary bodies included in the polysaccharide, at least one may be a modified monosaccharide unitary body. The modified monosaccharide unitary body means a unitary body into which a functional group that has not been present in the original unitary body is introduced through chemical treatment.

A polysaccharide in which such a modified polysaccharide unitary body exists may be called a modified

polysaccharide.

The polysaccharide component may comprise the modified polysaccharide.

In one example, the functional group introduced into the modified monosaccharide unitary body may be represented by, for example, Formula 1 below.

[Formula 1]

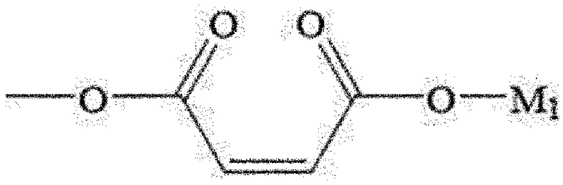

In Formula 1, $M_1$ is hydrogen or a metal, and when $M_1$ is the metal, the O-$M_1$ bond is an ionic bond.

The functional group of Formula 1 may be introduced into the unitary body by bonding the oxygen atom on the left side of Formula 1 above to the skeleton of the unitary body.

In the functional group of Formula 1, the carbon-carbon double bond may exist in the polymer in a state where the relevant double bond is maintained, or may exist in the polymer in a state where the double bond participates in cross-linking.

In Formula 1, $M_1$ is hydrogen or a metal. The type of metal is not particularly limited, which may usually be an alkali metal such as lithium, sodium, potassium, or cesium.

In the polysaccharide, a functional group where $M_1$ in Formula 1 above is hydrogen and a functional group where $M_1$ is a metal may also exist simultaneously.

The functional group may be introduced by reacting the polysaccharide with an unsaturated dicarboxylic acid or an anhydride thereof and substituting a hydroxy group present in the unitary body of the polymer with the functional group. An example of the dicarboxylic acid or anhydride thereof may be exemplified by maleic acid or maleic anhydride, but is not limited thereto, and salts of maleic acid may also be applied.

The lower limit of the substitutional rate of the functional group of Formula 1 above in the polysaccharide component may be 10%, 15%, 20%, 25%, 30%, 35%, 40%, 45%, 50%, 55%, 60%, 65%, 70%, 75%, 80%, 85%, 90%, or 95% or so, and the upper limit thereof may be 300%, 295%, 290%, 285%, 280%, 275%, 270%, 265%, 260%, 255%, 250%, 245%, 240%, 235%, 230%, 225%, 220%, 215%, 210%, 205%, 200%, 195%, 190%, 185%, 180%, 175%, 170%, 165%, 160%, 155%, 150%, 145%, 140%, 135%, 130%, 125%, 120%, 115%, 110%, 105%, 100%, 95%, 90%, 85%, 80%, 75%, 70%, 65%, 60%, 55%, 50%, 45%, or 40% or so. The substitutional rate may also be within a range of more than or equal to, or more than any one of the above-described lower limits; or within a range of less than or equal to, or less than any one of the above-described upper limits; or within a range of less than or equal to, or less than any one of the above-described upper limits while being more than or equal to, or more than any one of the above-described lower limits. Within the range of the substitutional rate, cross-linking of the polysaccharide component can proceed effectively, and the physical properties (for example, absorption capacity and/or biodegradability, etc.) of the resulting polymer material can be stably secured.

In this specification, the substitutional rate is a value indicating how much the hydroxy groups present in the respective unitary bodies included in the polysaccharide component are replaced with predetermined functional groups (e.g., functional groups of Formula 1 above), and is an average value of substituted degrees of the respective unitary bodies present in the polysaccharide. For example, if the unitary body is a grape sugar (glucose) unit, there are three hydroxy groups in the relevant unit before modification, and therefore, if all the hydroxy groups are substituted with the functional groups of Formula 1, the substitutional rate for the relevant unit is 300%. However, the substitutional rate of the polysaccharide is the average value of the substituted degrees of the respective units present in the polysaccharide, so that for example, when in a polysaccharide containing 5 grape sugar (glucose) units, the substitutional rates of the respective units are 100%, 0%, 200%, 300% and 100%, the substitutional rate of the polysaccharide is 140%, which is the average value. Such a substitutional rate can be identified through $^1$H NMR analyses of the polysaccharide. That is, since the hydroxy groups and substituted functional groups present in the polysaccharide can be quantified through the $^1$H NMR analysis, the substitutional rate can be identified, and if necessary, the substitutional rate can be calculated considering the $^1$H NMR analysis results of the polysaccharide before modification. In this way, the method of quantifying functional groups through the $^1$H NMR analysis is known.

In one example, the unitary body containing the functional group of Formula 1 may be a unitary body represented by Formula 2 below.

[Formula 2]

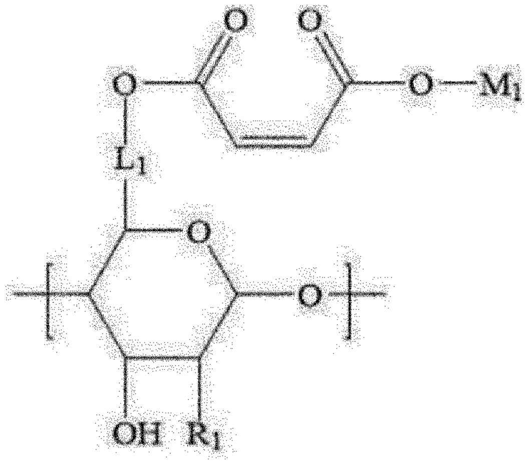

In Formula 2, $R_1$ is a hydroxy group, an amino group, or an alkylcarbonylamino group, $L_1$ is an alkylene group or an alkylidene group, and $M_1$ is hydrogen or a metal.

If $M_1$ in Formula 2 is the metal, the bond of O-$M_1$ in Formula 2 may be an ionic bond.

The specific type of the metal of $M_1$ in Formula 2 is as described in Formula 1.

In Formula 2, the specific type of the alkyl, alkylene group, or alkylidene group is the same as defined at the opening.

As described in Formula 1, the carbon-carbon double bond of the functional group in Formula 2 may exist in the polymer in a state where the double bond is maintained, or may exist in the polymer in a state where the double bond participates in cross-linking.

In the polymer, a functional group where $M_1$ in Formula 2 above is hydrogen and a functional group where $M_1$ is a metal may also exist simultaneously.

In Formula 2, when $R_1$ is a hydroxy group, the unitary body may usually be a case derived from a so-called grape sugar unitary body; when $R_1$ is an amino group, the unitary body may usually be a case derived from a so-called glucosamine unitary body; and when $R_1$ is an alkylcarbonylamino group, the unitary body may represent a case derived from N-acetylglucosamine.

In one example, the functional group introduced into the modified monosaccharide unitary body may also be, for example, a functional group represented by Formula 3 below.

[Formula 3]

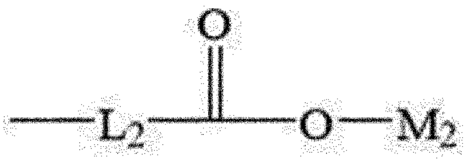

In Formula 3, $L_2$ is an alkylene group or an alkylidene group, $M_2$ is hydrogen or a metal, and when $M_2$ is the metal, the O-$M_2$ bond is an ionic bond.

In Formula 3, the specific type of the alkylene group or alkylidene group is the same as defined at the opening.

The functional group of Formula 3 may be introduced into the unitary body as $L_2$ on the left side in Formula 3 above is bonded to the skeleton of the unitary body.

In Formula 3, $M_2$ is hydrogen or a metal. The type of the metal is not particularly limited, which may usually be an alkali metal such as lithium, sodium, potassium, or cesium.

In the polymer, a functional group where $M_2$ in Formula 3 above is hydrogen and a functional group where $M_2$ is a metal may also exist simultaneously.

The functional group can be introduced by reacting the polysaccharide with acetic acid chloride such as chloroacetate and substituting the hydroxy group present in the unitary body of the polymer with the functional group.

The lower limit of the substitutional rate of the functional group of Formula 3 in the polysaccharide component

may be 10%, 15%, 20%, 25%, 30%, 35%, 40%, 45%, 50%, 55%, 60%, 65%, 70%, 75%, 80%, 85%, 90%, or 95% or so, and the upper limit thereof may be 300%, 295%, 290%, 285%, 280%, 275%, 270%, 265%, 260%, 255%, 250%, 245%, 240%, 235%, 230%, 225%, 220%, 215%, 210%, 205%, 200%, 195%, 190%, 185%, 180%, 175%, 170%, 165%, 160%, 155%, 150%, 145%, 140%, 135%, 130%, 125%, 120%, 115%, 110%, 105%, 100%, 95%, 90%, 85%, 80%, 75%, or 70% or so. The substitutional rate may also be within a range of more than or equal to, or more than any one of the above-described lower limits; or within a range of less than or equal to, or less than any one of the above-described upper limits; or within a range of less than or equal to, or less than any one of the above-described upper limits while being more than or equal to, or more than any one of the above-described lower limits. When the functional group of Formula 3 exists, within the range of the substitutional rate, cross-linking of the polysaccharide component can proceed effectively, and the physical properties (for example, absorption capacity and/or biodegradability, etc.) of the resulting polymer material can be stably secured.

The definition of the substitutional rate is the same as defined for the functional group in Formula 1, which can be identified through the $^1$H NMR analysis of the polysaccharide component equally.

In one example, the unitary body containing the functional group of Formula 3 above may be a unitary body represented by Formula 4 below.

[Formula 4]

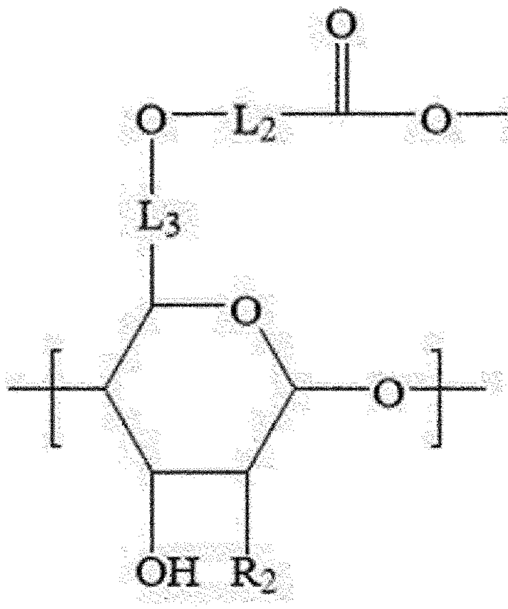

In Formula 4, $R_2$ is a hydroxy group, an amino group, or an alkylcarbonylamino group, $L_2$ and $L_3$ are each independently an alkylene group or an alkylidene group, and $M_2$ is hydrogen or a metal.

When $M_2$ above is the metal, the O-$M_2$ bond is an ionic bond.

The specific type of the metal of $M_2$ in Formula 4 is as described in Formula 3.

In Formula 4, the specific type of the alkyl, alkylene group, or alkylidene group is the same as defined at the opening.

In the polysaccharide, a functional group in which $M_2$ in Formula 4 above is hydrogen and a functional group in which $M_2$ is a metal may also exist simultaneously.

In the polysaccharide included in the polymer composition, only one of the functional group of Formula 1 above and the functional group of Formula 3 may exist, or both may also exist simultaneously.

Therefore, in the polysaccharide component of the polymer composition, any one of the unitary body of Formula 2 above and the unitary body of Formula 4 may exist, or both may also exist.

In one example, the polysaccharide may be included in a cross-linked state in the polymer composition. Therefore, the polymer composition may further comprise a cross-linking agent. However, if the cross-linking of the polymer composition is by self-cross-linking to be described below, the polymer composition does not necessarily need to include a cross-linking agent.

In one example, the cross-linked state of the polysaccharide component may be a self-cross-linked state. The term self-cross-linking may mean a case where polysaccharides within a polysaccharide component are cross-linked without application of a separate cross-linking agent, or a case where cross-linking is achieved using a minimal cross-linking agent.

Through cross-linking in this manner, it is possible to provide a polymer composition with excellent absorption capacity and biodegradability at the same time. In general, polysaccharides have a low cross-linking efficiency, so that efficient cross-linking is not achieved even when cross-linking functional groups are introduced, but in the case of the present application, the self-cross-linking can be effectively performed through application of the specific polysaccharide as described above.

When the polysaccharide component is self-cross-linked, the upper limit of the weight ratio of the cross-linking agent relative to 100 parts by weight of the polysaccharide component in the polymer composition may be 10 parts by weight, 9 parts by weight, 8 parts by weight, 7 parts by weight, 6 parts by weight, 5 parts by weight, 4 parts by weight, 3 parts by weight, 2 parts by weight, 1 part by weight, 0.5 parts by weight, 0.1 parts by weight, 0.05 parts by weight, 0.01 parts by weight, 0.005 parts by weight, or 0.001 parts by weight or so, and the lower limit thereof may be about 0 parts by weight or so. The content may also be within a range of more than or equal to, or more than any one of the above-described lower limits; or within a range of less than or equal to, or less than any one of the above-described upper limits while being more than or equal to, or more than any one of the above-described lower limits. The cross-linking achieved by using the cross-linking agent in the above ratio can also be regarded as cross-linking that falls into the category of self-cross-linking.

The method of performing the self-cross-linking is not particularly limited. The self-cross-linking may be performed, for example, in the presence of an oxidizing agent such as ammonium persulfate, or other initiators.

For example, when the functional group of Formula 1 above is present in the polysaccharide component, in the cross-linking environment as above, cross-linking or polymerization by the functional group of Formula 1 present in the polysaccharide, and cross-linking or polymerization, or grafting by the radicals generated by oxidation by the oxidizing agent and the functional group of Formula 1 above may proceed to perform cross-linking.

In another example, the cross-linking may also be performed using a cross-linking agent. In this case, as the cross-linking agent, a cross-linking agent applied as a so-called internal cross-linking agent or a cross-linking agent applied as an external cross-linking agent in the production of cross-linked polyacrylic acid, which is usually applied as a so-called SAP (superabsorbent polymer), without any special limitation may be applied, and both the foregoing may also be applied.

As such a cross-linking agent, polyethylene glycol diacrylate, N,N'-methylenebisacrylamide, trimethylolpropane tri(meth)acrylate, ethylene glycol di(meth)acrylate, polyethylene glycol (meth)acrylate, propylene glycol di(meth)acrylate, polypropylene glycol (meth)acrylate, butanediol di(meth)acrylate, butylene glycol di(meth)acrylate, diethylene glycol di(meth)acrylate, hexane diol (meth)acrylate, triethylene glycol di(meth)acrylate, tripropylene glycol di(meth)acrylate, tetraethylene glycol di(meth)acrylate, dipentaerythritol pentaacrylate, glycerin tri(meth)acrylate, pentaerythol tetraacrylate, triarylamine, ethylene glycol diglycidyl ether, propylene glycol, glycerin and/or ethylene carbonate, and the like are known, and in the present application, if necessary, an appropriate cross-linking agent may be selected from among the known cross-linking agents, and used.

For performing more effective cross-linking and securing desired physical properties, as the cross-linking agent, an organic acid having two or more carboxyl groups or an anhydride of the organic acid may be applied in addition to the cross-linking agent.

When the cross-linking agent is applied, an ester bond formed by reacting the carboxyl group of the cross-linking agent with the hydroxy group in the polysaccharide component may be present in the polymer composition.

At this time, the type of the organic acid is not particularly limited, but for example, an organic acid having a molecular weight (molar mass) in a range of about 90 to 300 g/mol or about 100 to 250 g/mol may be used. The lower limit of the number of carboxyl groups contained in the organic acid may be 2, or 3 or so, and the upper limit thereof may be 10, 9, 8, 7, 6, 5, 4, or 3 or so. The number of carboxyl groups may also be within a range of more than or equal to, or more than any one of the above-described lower limits or within a range of less than or equal to, or less than any one of the above-described upper limits while being more than or equal to, or more than any one of the above-described lower limits.

Such an organic acid may be exemplified by citric acid, succinic acid, pimelic acid, or adipic acid, but is not limited thereto. The organic acid or its anhydride may be used as the cross-linking agent.

When the cross-linking agent is used, the upper limit of the weight ratio of the cross-linking agent relative to 100 parts by weight of the polysaccharide component may be 10 parts by weight, 9 parts by weight, 8 parts by weight, 7 parts by weight, 6 parts by weight, 5 parts by weight, 4 parts by weight, or 3 parts by weight or so, and the lower limit thereof may be 0.5 parts by weight, 1 part by weight, 1.5 parts by weight, 2 parts by weight, 2.5 parts by weight, or 3 parts by weight or so. The content may also be within a range of more than or equal to, or more than any one of the above-described lower limits; or within a range of less than or equal to, or less than any one of the above-described upper limits; or within a range of less than or equal to, or less than any one of the above-described upper limits while being more than or equal to, or more than any one of the above-described lower limits. The content range of the cross-linking agent may also be a combination of any one value of the above-described upper limits and any one value of the above-described lower limits.

To the polymer composition, additional treatments, for example, surface cross-linking treatment or grinding treatment, and the like may also be performed. In this case, the polymer composition may comprise the polysaccharide component in the form of particles while being in a cross-linked state, or may comprise the polysaccharide component, in

which a surface cross-linking agent is coupled to the surface, while being in a cross-linked state and in the form of particles.

The polymer composition can be used for various applications by exhibiting excellent absorptivity and biodegradability simultaneously.

For example, the polymer composition may be used as sanitary products such as diapers or sanitary napkins, or absorbent materials applied to other applications requiring absorption. If necessary, further cross-linking, surface treatment, or physical grinding processes may also be performed on the polymer composition to increase the efficiency for use as the sanitary products or absorbent materials.

Therefore, the present application relates to an absorbent material or sanitary product (e.g. diapers, sanitary napkins, etc.) comprising the polymer composition.

The specific method of forming the absorbent material or sanitary product by applying the polymer composition is not particularly limited, and for example, the method of forming the absorbent material or sanitary product by applying the existing SAP may be used in the same manner.

## Advantageous Effects

The present application can provide a polymer composition having excellent biodegradability and absorption capacity, and a use thereof.

## Description of Drawings

Figures 1 to 5 are views showing $^1H$ NMR analysis results of polysaccharides prepared in Preparation Examples.

## Mode for Invention

Hereinafter, the present application will be described in detail through examples and comparative examples, but the scope of the present application is not limited by the following examples.

### 1. Evaluation of centrifuge retention capacity (CRC)

Centrifuge retention capacity (CRC) was measured according to EDANA (European Disposables and Nonwovens Association) WSP 241.3. About 0.2 g ($W_0$) of a polymer composition was placed in a non-woven bag, sealed, and then submerged in a physiological saline solution. As the physiological saline solution, an aqueous NaCl solution with a concentration of 0.9 wt% was used. The state was maintained for 30 minutes or so, water was removed from the bag for 3 minutes under a condition of 250 G using a centrifuge, and the mass (g, $W_2$) of the bag was measured. The same operation was performed on the same non-woven bag containing no polymer composition, and the mass (g, $W_1$) was measured.

The CRC (g/g) was calculated by substituting the measurement results into Equation A below.

The evaluation was conducted under constant temperature and humidity conditions (23 ± 1°C, relative humidity: 50 ± 10%).

[Equation A]

$$CRC\ (g/g) = \{[W_2(g) - W_1(g)]/W_0(g)\} - 1$$

### 2. Evaluation of absorption capacity under pressure (AUP)

Absorption capacity under pressure (AUP, 0.7 psi) of a polymer composition was measured according to the standard of EDANA (European Disposables and Nonwovens Association) WSP 242.3. A 400-mesh stainless steel wire net was mounted on the bottom of a plastic cylinder with an inner diameter of 60 mm or so. Under conditions of a temperature of 23 ± 2°C and a relative humidity of 50%, about 0.90 g ($W_0$) of the polymer composition was uniformly sprayed on the wire net, and a piston capable of further uniformly imparting a load of about 0.7 psi was installed thereon to manufacture a measuring device. As the piston, a piston with an outer diameter slightly smaller than 60 mm was used, which was installed so that it could move up and down without forming a gap with the inner wall of the cylinder. The weight (unit: g) ($W_3$) of the measuring device was measured.

A glass filter with a diameter of 90 mm or so and a thickness of 5 mm or so was placed on the inside of a petro dish with a diameter of about 150 mm, and a physiological saline solution (NaCl aqueous solution with a concentration of 0.9 wt%) was applied to be at the same level as the upper surface of the glass filter. One sheet of filter paper with a diameter of 90 mm or so was placed thereon. The measuring device was placed on the filter paper and the physiological saline solution

was absorbed for 1 hour under a load of 0.7 psi. After 1 hour, the measuring device was lifted, and the weight $W_4$ (g) was measured.

The absorption capacity under pressure (AUP) (g/g) was calculated by substituting the respective weights as measured into Equation B below.

[Equation B]

$$AUP\ (g/g) = [W_4(g) - W_3(g)]/W_0(g)$$

**3. Measurement of biodegradability**

Biodegradability was measured in the manner specified in ISO 14855-1 (2005) standard. The above standard is a method of measuring aerobic biodegradability of plastic materials under a composting condition, which is a method that the biodegradability of a polymer composition is calculated by quantifying the amount of carbon dioxide emitted by microorganisms metabolizing the relevant material. As the polymer composition was applied to the composting condition according to the standard, the biodegradability was measured for 6 months, and the biodegradability was obtained as the ratio of the theoretical carbon dioxide generation amount and the actual carbon dioxide generation amount of the material. Here, the theoretical carbon dioxide generation amount and biodegradability are obtained according to the following equations C and D, respectively.

$$\text{Theoretical carbon dioxide generation amount (ThCO2, g/container)} = MTOT \times CTOT \times (44/12) \quad \text{[Equation C]}$$

In Equation C, MTOT is the amount (g) of total dry solid content in the test material (polymer composition) added to the compost at the start of the measurement, and CTOT means the ratio (g/g) of organic carbon contained in the total dry solid content of the test material.

[Equation D]

$$\text{Biodegradability (\%)} = [\{(CO2)T-(CO2)B\}/ThCO2] \times 100$$

In Equation D, (CO2)T is the accumulated amount (g/container) of carbon dioxide generated from the composting container containing the test material, (CO2)B is the average (g/container) of carbon dioxide accumulated amounts generated from the inoculum source container, and ThCO2 is the theoretical carbon dioxide generation amount confirmed in Equation C above.

**4. Molecular weight measurement of polysaccharide component**

A molecular weight of a polysaccharide component was evaluated in the following manner.

(1) Preparation of mobile phase

A mobile phase A was prepared by filtering 1000 mL of a 150 mM $NaNO_3$ aqueous solution containing 0.02 wt% of $NaN_3$ using a solvent clarification system (Millipore Millisolve Kit, MilliporeSigma).

(2) Preparation of sample solution

A sample intended to be measured was collected in an amount of 25 mg, and mixed with 5 mL of a 150 mM $NaNO_3$ aqueous solution containing 0.02 wt% of $NaN_3$, and then a sample solution was prepared by heating the mixture at 80°C for 20 hours, and then filtering it with a 0.4μm Nylon Syringe Filter.

(3) GPC (Gel Permeation Chromatography)/MALS (Multi-Anglue Light Scattering Detection) conditions

The molecular weight was evaluated using the sample solution and the mobile phase A in the following manner.

Measuring instrument: Agilent GPC (Agilent 1200 series, U.S.)

Stationary phase: connecting Shodex OH-Pak 804 column and Shodex OH-Pak 80 column

Mobile phase: A; 0.02% $NaN_3$, 150 mM $NaNO_3$ aqueous solution = 100 (v/v %)

Flow rate: 0.4 mL/min

Stationary phase temperature: 25°C

Injected amount: 100 μl (0.45 μm filtered)

Analysis time: 120 minutes

**5. Content measurement of amylopectin and amylose in polysaccharide component**

Contents of amylopectin and amylose in a polysaccharide component were evaluated according to the method described in a paper (Potato Research 31 (1988) 241-246).

First, a sample was prepared by dissolving about 5 mg of a polysaccharide (starch) in about 1 mL of sterile water (Step 1), and heated to 95°C for about 15 minutes in a constant temperature water bath (Step 2).

Subsequently, about 20 μl of the sample was placed in a cuvette (Step 3), and about 980μl of an iodine solution was added thereto and mixed (Step 4).

Subsequently, absorbance of the sample mixed with the iodine solution at wavelengths of 525 nm and 700 nm was measured and recorded, respectively (Step 5). The absorbance was measured using KLAB's OPTIZEN POP model.

About 20 μl of water was placed in another cuvette, 980 μl of the iodine solution was added thereto, and mixed (Step 6). For the solution of Step 6, absorbance at wavelengths of 525 nm and 700 nm was measured and recorded, respectively, in the same manner as in Step 5 (Step 7).

The absorbance obtained in Step 7 was subtracted from the absorbance obtained in Step 5, and the ratio (%) of amylose was confirmed according to Equation E below (Step 8).

[Equation E]

$$PA = 3.039 - \frac{7.154 \times \frac{OD_{700}}{OD_{525}}}{3.048 \times \frac{OD_{700}}{OD_{525}}} - 19.192$$

In Equation E, PA is the ratio (%) of amylose, $OD_{700}$ is the value obtained by subtracting the absorbance at a wavelength of 700 nm measured in Step 7 from the absorbance at a wavelength of 700 nm measured in Step 5, and $OD_{525}$ is the value obtained by subtracting the absorbance at a wavelength of 525 nm measured in Step 7 from the absorbance at a wavelength of 525 nm measured in Step 5.

Preparation Example 1.

Starch (Compound A) containing a modified monosaccharide unitary body of Formula A below was prepared in the following manner. The modified monosaccharide unitary body of Formula A below is a monosaccharide unitary body into which a functional group derived from sodium acetate has been introduced.

[Formula A]

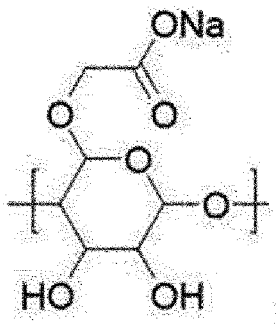

As the starch material, starch with a weight average molecular weight (Mw) of about 180,000,000 g/mol or so, an amylopectin ratio of about 81%, and an amylose ratio of about 19% was used. The ratio of amylose is the value confirmed by Equation A above, and the ratio of amylopectin is the value obtained by subtracting the ratio of amylose from 100%.
The starch, isopropyl alcohol (IPA), and NaOH aqueous solution (concentration: about 40%) were mixed in a 500 mL RBF (Round Bottom Flask), and stirred at room temperature for about 20 minutes or so. The weight ratio of the mixing was set to 1:4:0.8 (starch: IPA: NaOH aqueous solution). Subsequently, about 80 parts by weight of sodium monochloroacetate was added thereto relative to 100 parts by weight of the starch, the temperature was raised to 60°C, and then stirred for 1 hour or so, and the temperature was cooled to room temperature. After cooling to room temperature, the solvent was removed from water, the reactant was dissolved in water, precipitated in methanol to remove water-soluble impurities, filtered to recover starch, and dried in a vacuum drying oven at 40°C for about 12 hours to obtain a solid target product (Compound A).
The substitutional rate of the obtained target product (Compound A) can be obtained through a $^1$H NMR analysis. The $^1$H NMR analysis is performed at room temperature using a $^1$H NMR spectrometer including a Varian Unity Inova (500 MHz) spectrometer with a triple resonance 5 mm probe. In the $^1$H NMR analysis, Bruker's Avance Neo instrument was used.
Specifically, 50 mg of the obtained solid target product (Compound A) and 200 mg of 30% D2DSO4 in D2O solution are mixed, stirred at 50°C for 1 hour or so to induce a hydrolysis reaction, and then the $^1$H NMR analysis can be performed.
The $^1$H NMR analysis results of the target product (Compound B) are shown in Figure 1. The substitutional rate confirmed by the above analysis was about 60% or so.

**Preparation Example 2.**

Modified starch was prepared in the same manner as in Preparation Example 1, except that as the starch, starch with a weight average molecular weight (Mw) of about 12,000,000 g/mol or so, an amylopectin ratio of about 75%, and an amylose ratio of about 25% was used.
The $^1$H NMR results of the starch identified in the same manner as Preparation Example 1 are the same as in Figure 2 below, and the substitutional rate confirmed from the results was about 60% or so.

**Preparation Example 3.**

Modified starch was prepared in the same manner as in Preparation Example 1, except that as the starch, starch with a weight average molecular weight (Mw) of about 2,000,000 g/mol or so, an amylopectin ratio of about 69%, and an amylose ratio of about 31% was used.
The $^1$H NMR results of the starch identified in the same manner as in Preparation Example 1 are the same as in Figure 3, and the substitutional rate confirmed from the results was about 70% or so.

**Preparation Example 4.**

Modified starch was prepared in the same manner as in Preparation Example 1, except that as the starch, starch with a weight average molecular weight (Mw) of about 920,000 g/mol or so, an amylopectin ratio of about 58%, and an amylose ratio of about 42% was used.
The $^1$H NMR results of the starch identified in the same manner as in Preparation Example 1 are the same as in Figure 4, and the substitutional rate confirmed from the results was about 52% or so.

**Preparation Example 5.**

Modified starch was prepared in the same manner as in Preparation Example 1, except that as the starch, starch with a weight average molecular weight (Mw) of about 450,000 g/mol or so, an amylopectin ratio of about 19%, and an amylose ratio of about 81% was used.

The $^1$H NMR results of the starch identified in the same manner as in Preparation Example 1 are the same as in Figure 5, and the substitutional rate confirmed from the results was about 50% or so.

**Preparation Example 6.**

Modified starch was prepared in the same manner as in Preparation Example 1, except that as the starch, starch with a weight average molecular weight (Mw) of about 140,000 g/mol or so, an amylopectin ratio of about 6%, and an amylose ratio of about 94% was used.

As a result of $^1$H NMR of the starch identified in the same manner as in Preparation Example 1, the substitutional rate was about 60% or so.

**Example 1.**

The starch prepared in Preparation Example 1 was dissolved in distilled water, and a cross-linking agent (citric acid) was added at a ratio of about 3 parts by weight relative to 100 parts by weight of the starch. It was stirred at room temperature for 30 minutes or so, transferred to a steel tray, and dried in an oven at about 40°C or so for 12 hours or so. Subsequently, in a state where the temperature was raised to about 80°C or so, it was cross-linked for 2 hours or so to prepare a desired polymer composition.

**Example 2.**

A polymer composition was prepared in the same manner as in Example 1, except that the starch prepared in Preparation Example 2 was used.

**Example 3.**

A polymer composition was prepared in the same manner as in Example 1, except that the starch prepared in Preparation Example 3 was used.

**Example 4.**

A polymer composition was prepared in the same manner as in Example 1, except that the starch prepared in Preparation Example 4 was used.

**Example 5.**

A polymer composition was prepared in the same manner as in Example 1, except that the starch prepared in Preparation Example 5 was used.

**Comparative Example 1.**

A polymer composition was prepared in the same manner as in Example 1, except that the starch prepared in Preparation Example 6 was used.

The Mw, Am, and Ap values, and F values of the polymer compositions of Examples and Comparative Example in Equation 1, as well as the CRC, AUP, and biodegradability measured for each polymer composition were summarized and described in Table 1 below. In Table 1 below, the unit of Mw is × 1,000 g/mol.

[Table 1]

| | Example | | | | | Comparative Example |
|---|---|---|---|---|---|---|
| | 1 | 2 | 3 | 4 | 5 | 1 |
| F | 8.2 | 7.0 | 6.1 | 5.7 | 4.9 | 3.9 |

(continued)

| | Example | | | | | Comparative Example |
|---|---|---|---|---|---|---|
| | 1 | 2 | 3 | 4 | 5 | 1 |
| Mw | 180,000 | 12,000 | 2,000 | 920 | 450 | 140 |
| Ap (%) | 81 | 75 | 69 | 58 | 19 | 6 |
| Am (%) | 19 | 25 | 31 | 42 | 81 | 94 |
| CRC (g/g) | 45.3 | 44.3 | 39.5 | 34.3 | 30.5 | 11.7 |
| AUP (g/g) | 5.51 | 4.73 | 3.59 | 4.52 | 4.21 | 1.62 |
| Biodegradability (%) | 75.7 | 85.8 | 94.2 | 99.3 | 99.5 | 99.5 |

From the results of Table 1, it can be confirmed that the polymer compositions in which F in Equation 1 is 4 or more simultaneously exhibit excellent absorption properties and biodegradability, whereas in the case of Comparative Example 1 in which the F is 3.9, biodegradability has been secured, but absorption properties are significantly reduced.

## Claims

1. A polymer composition comprising a polysaccharide component including amylose and amylopectin, wherein

   the polysaccharide component has F of 4 or more in Equation 1 below:

   [Equation 1]

   $$F = Log(Mw \times \frac{Ap}{Ap+Am})$$

   wherein, Ap is the ratio of the amylopectin in the polysaccharide component, Am is the ratio of the amylose in the polysaccharide component, and Mw is the weight average molecular weight of the polysaccharide component.

2. The polymer composition according to claim 1, having a centrifuge retention capacity of 12 g/g or more according to EDANA (European Disposables and Nonwovens Association) method WSP 241.3.

3. The polymer composition according to claim 1, having an absorption capacity under pressure at 0.7 psi of 1.5 g/g or more according to EDANA (European Disposables and Nonwovens Association) method WSP 242.3.

4. The polymer composition according to claim 1, having biodegradability of 60% or more.

5. The polymer composition according to claim 1, wherein the polysaccharide component comprises one or more selected from the group consisting of starch, dextrin, and chitosan.

6. The polymer composition according to claim 1, wherein Mw in Equation 1 is in a range of 200,000 g/mol to 1,000,000,000 /mol.

7. The polymer composition according to claim 1, wherein Ap in Equation 1 is in a range of 5% to 95%.

8. The polymer composition according to claim 1, wherein the polysaccharide component has a polysaccharide containing a functional group of Formula 1 below:

[Formula 1]

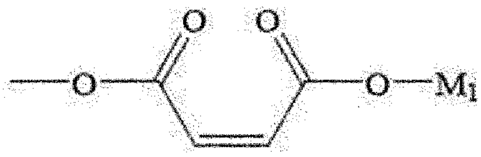

wherein, $M_1$ is hydrogen or a metal, and when $M_1$ above is the metal, the O-$M_1$ bond is an ionic bond, and the carbon-carbon double bond of Formula 1 may exist in a state of participating in the cross-linking.

**9.** The polymer composition according to claim 1, wherein the polysaccharide component has a polysaccharide containing a unitary body of Formula 2 below:

[Formula 2]

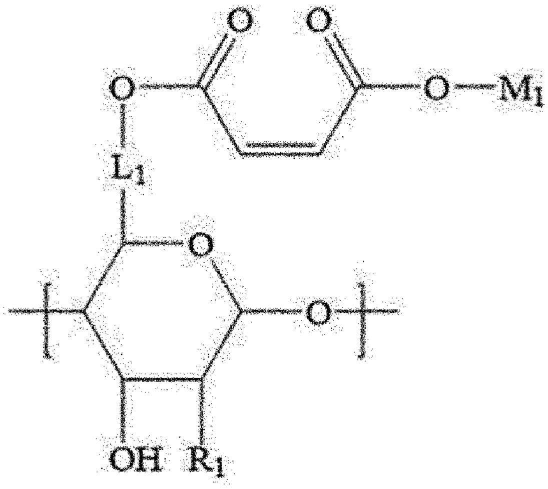

wherein, $M_1$ is hydrogen or a metal, and when $M_1$ above is the metal, the O-$M_1$ bond is an ionic bond, and the carbon-carbon double bond of Formula 2 may exist in a state of participating in the cross-linking, and $R_1$ is a hydroxy group, an amino group, or an alkylcarbonylamino group, and $L_1$ is an alkylene group or an alkylidene group.

**10.** The polymer composition according to claim 1, wherein the polysaccharide component has a polysaccharide containing a functional group of Formula 3 below:

[Formula 3]

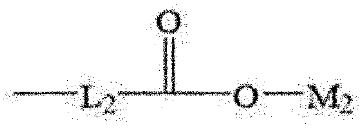

wherein, $L_2$ is an alkylene group or an alkylidene group, $M_2$ is hydrogen or a metal, and when $M_2$ above is the metal, the O-$M_2$ bond is an ionic bond.

**11.** The polymer composition according to claim 1, wherein the polysaccharide component has a polysaccharide containing a unitary body of Formula 4 below:

[Formula 4]

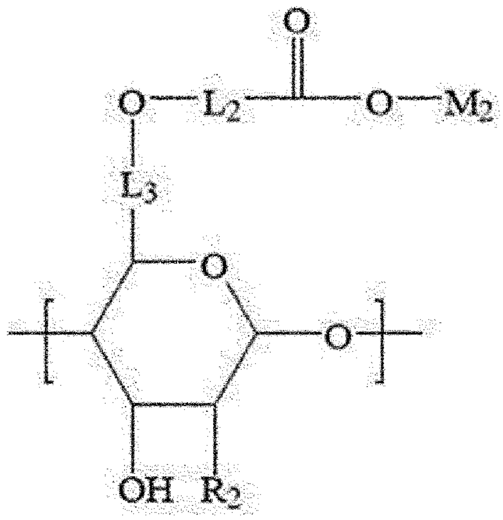

wherein, $R_2$ is a hydroxy group, an amino group, or an alkylcarbonylamino group, $L_2$ and $L_3$ are each independently an alkylene group or an alkylidene group, $M_2$ is hydrogen or a metal, and when $M_2$ above is a metal, the O-$M_2$ bond is an ionic bond.

**12.** The polymer composition according to claim 1, wherein the polysaccharide component is cross-linked.

**13.** The polymer composition according to claim 12, comprising an ester bond formed by reaction between the hydroxy group of the polysaccharide component and a cross-linking agent.

**14.** The polymer composition according to claim 12, wherein the polysaccharide component is cross-linked with a cross-linking agent, and the cross-linking agent is an organic acid containing two or more carboxyl groups, or an anhydride of the organic acid.

**15.** The polymer composition according to claim 14, wherein the organic acid has a molecular weight in a range of 90 g/mol to 300 g/mol, and contains 2 to 10 carboxyl groups.

**16.** The polymer composition according to claim 14, wherein the cross-linking agent is citric acid, citric anhydride, pimelic acid, pimelic anhydride, adipic acid, adipic anhydride, succinic acid, or succinic anhydride.

**17.** The polymer composition according to claim 14, comprising 0.5 parts by weight to 10 parts by weight of the cross-linking agent relative to 100 parts by weight of the polysaccharide component.

**18.** An absorbent material comprising the polymer composition of any one of claims 1 to 17.

**19.** A sanitary article comprising the polymer composition of any one of claims 1 to 17.

[Figure 1]

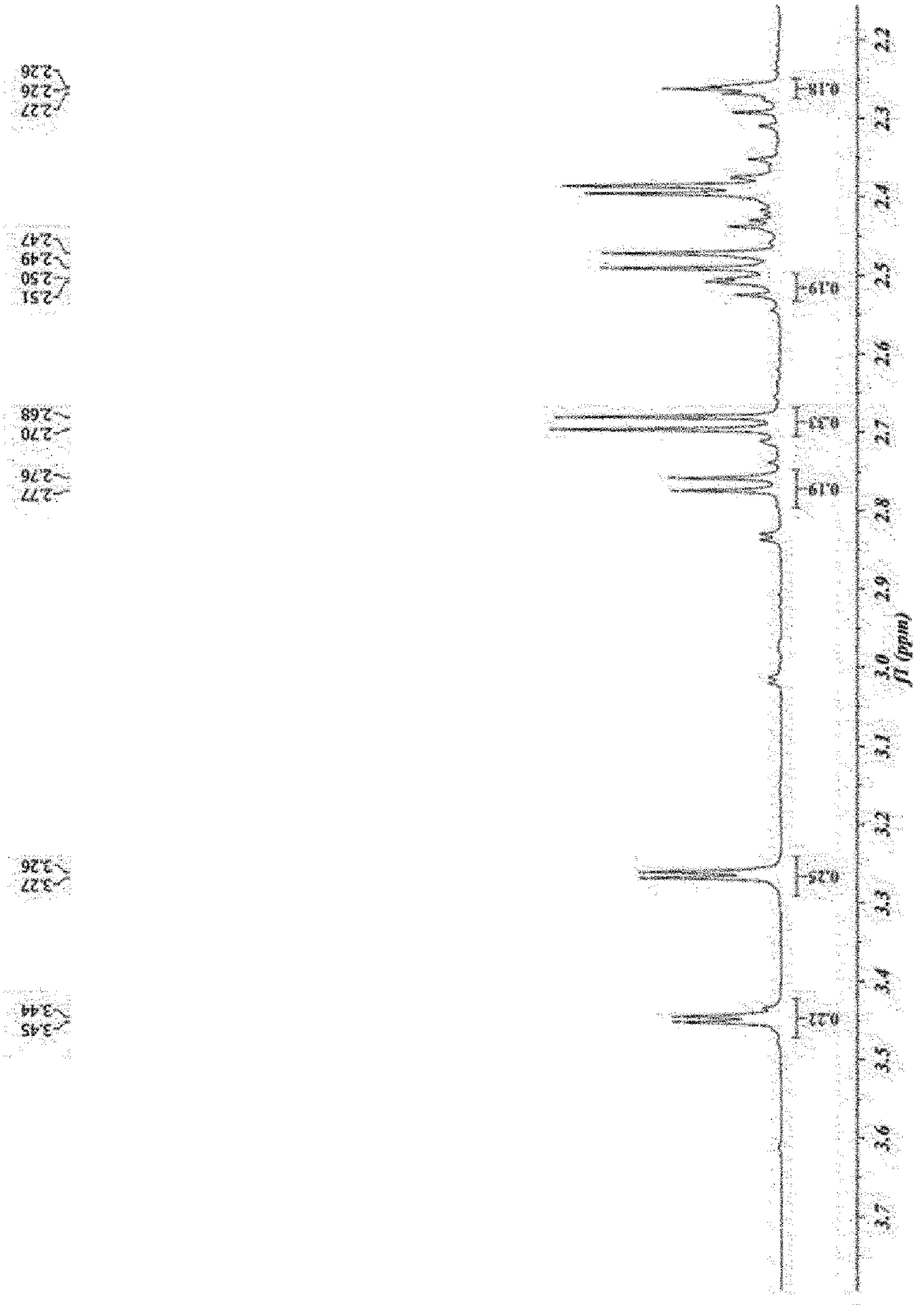
2.26
2.26
2.27
2.47
2.49
2.50
2.51
2.68
2.70
2.76
2.77
3.26
3.27
3.44
3.45
0.18
0.19
0.23
0.19
0.25
0.22
2.2
2.3
2.4
2.5
2.6
2.7
2.8
2.9
3.0
3.1
3.2
3.3
3.4
3.5
3.6
3.7
f1 (ppm)

[Figure 2]

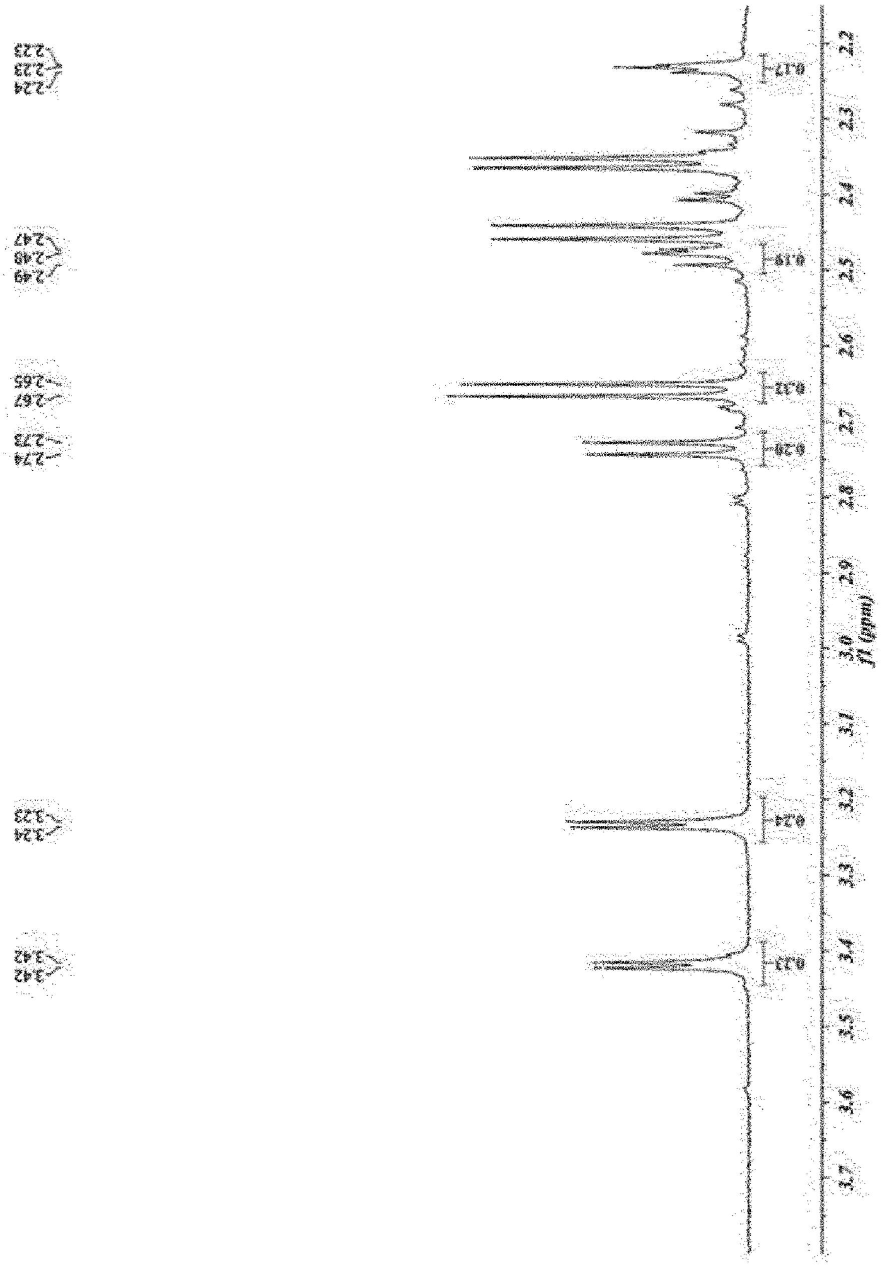

[Figure 3]

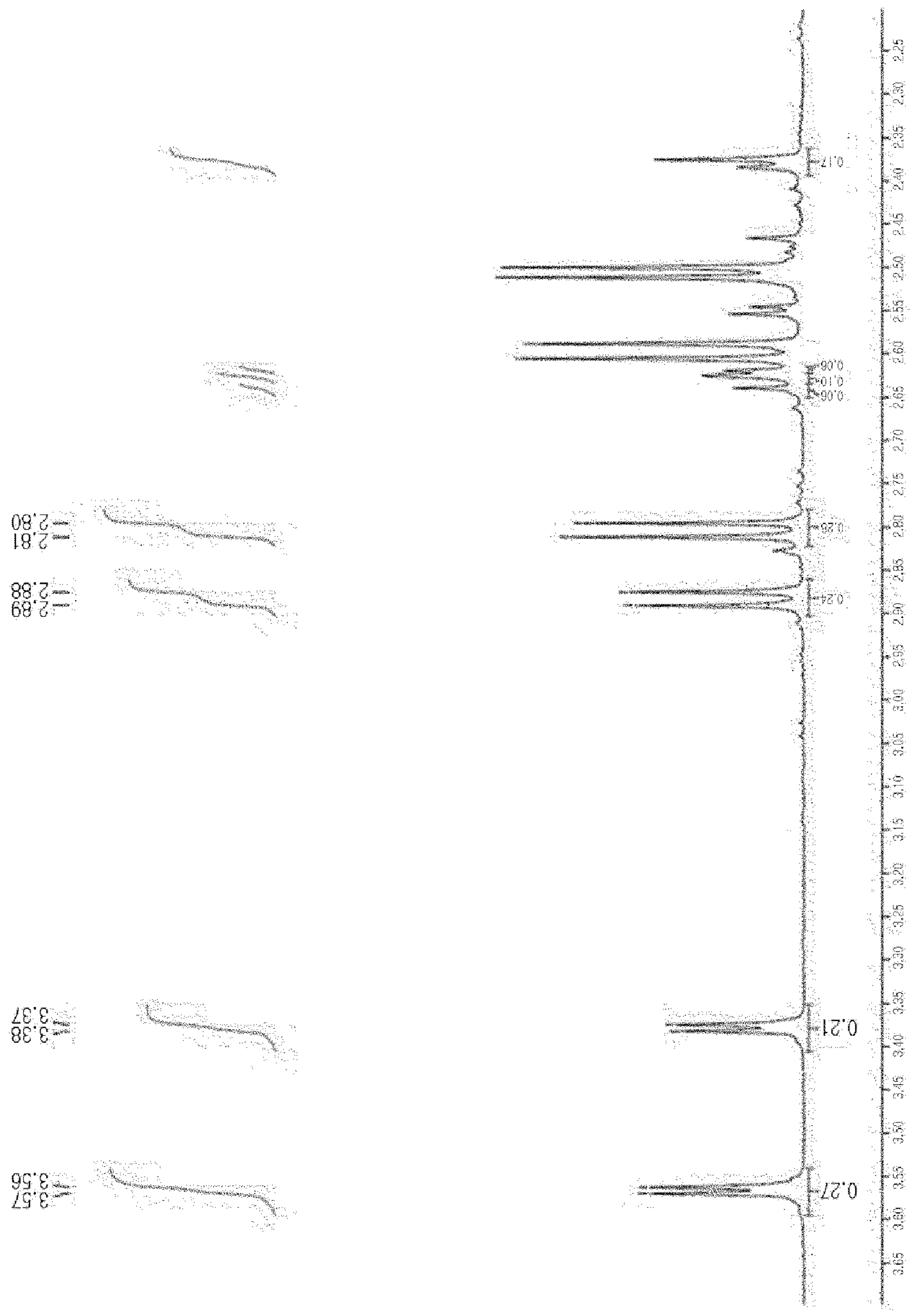

[Figure 4]

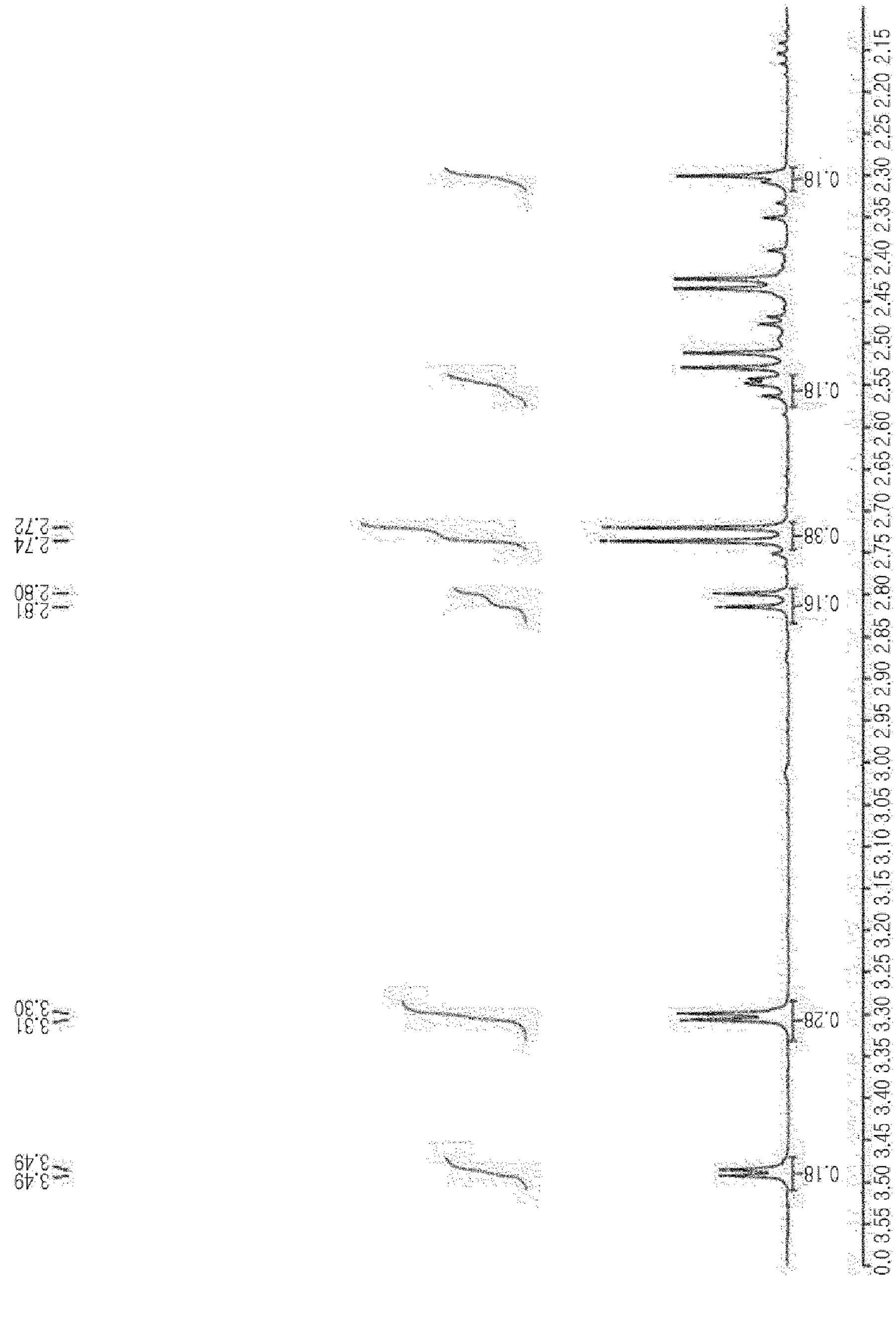

[Figure 5]

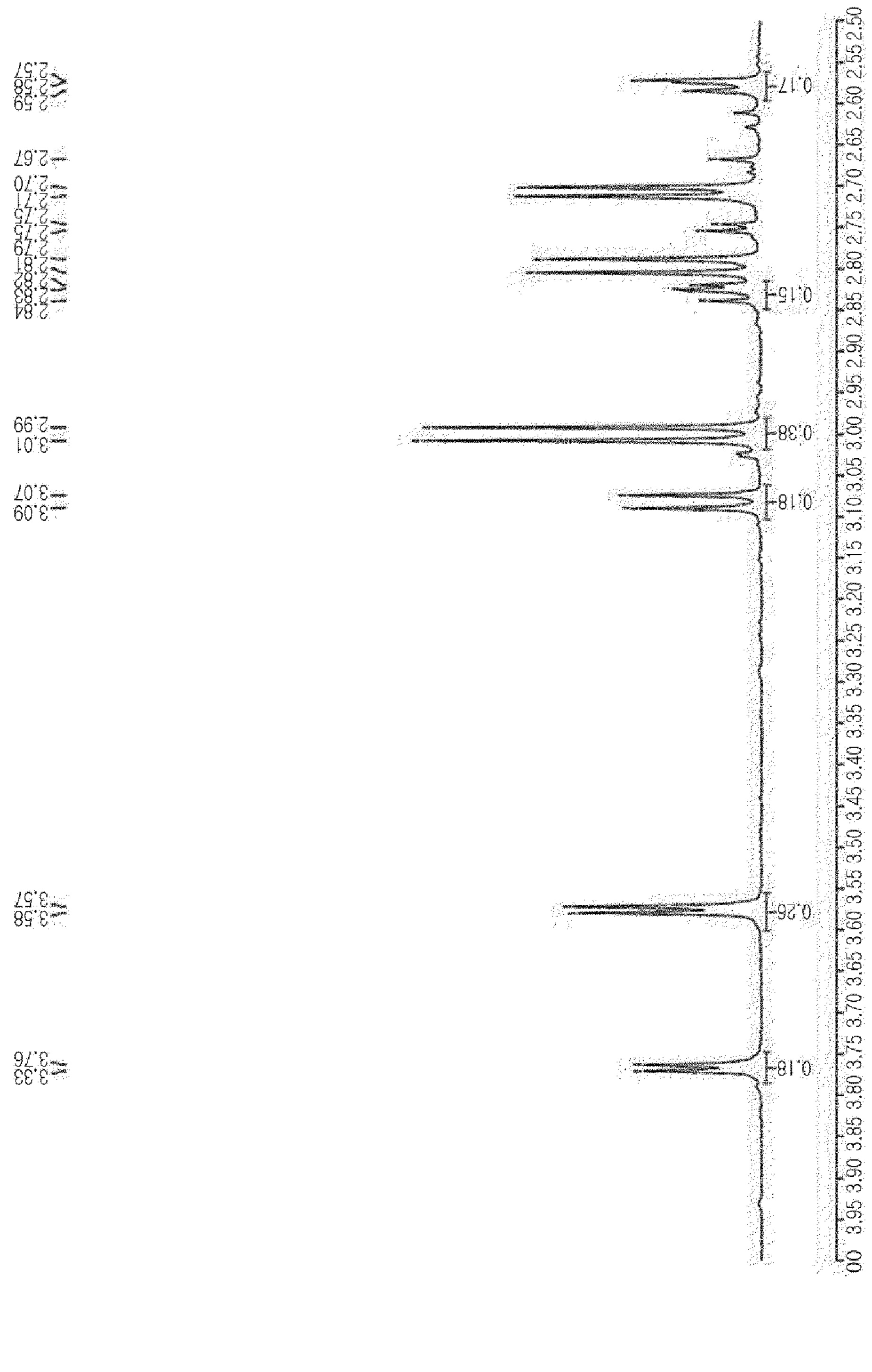

## INTERNATIONAL SEARCH REPORT

International application No.
**PCT/KR2023/013094**

**A. CLASSIFICATION OF SUBJECT MATTER**

**C08L 3/12**(2006.01)i

According to International Patent Classification (IPC) or to both national classification and IPC

**B. FIELDS SEARCHED**

Minimum documentation searched (classification system followed by classification symbols)

C08L 3/12(2006.01); A61L 15/60(2006.01); C08B 37/00(2006.01); C08F 220/20(2006.01); C08F 220/56(2006.01); C08F 8/00(2006.01); C08J 5/18(2006.01); C08L 23/06(2006.01); C08L 3/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 전분(starch), 아밀로오스(amylose), 아밀로펙틴(amylopectin), 초흡수성 폴리머(super absorbent polymer), 다당류(polysaccharide), 분자량(molecular weight), 푸마르산(fumaric acid), 시트르산(citric acid), 카르복시메틸기(carboxymethyl group), 가교(crosslink), 생분해성(biodegradability)

**C. DOCUMENTS CONSIDERED TO BE RELEVANT**

| Category* | Citation of document, with indication, where appropriate, of the relevant passages | Relevant to claim No. |
|---|---|---|
| X | KR 10-2020-0132929 A (MJJ TECHNOLOGIES INC.) 25 November 2020 (2020-11-25)<br>See paragraphs [0028], [0029], [0032], [0033], [0039]-[0041], [0043], [0045], [0046], [0053], [0144]-[0148] and [0151]-[0154]. | 1-7,10-12,18,19 |
| Y | | 13-17 |
| A | | 8,9 |
| Y | US 5550189 A (QIN, Jian et al.) 27 August 1996 (1996-08-27)<br>See column 5, lines 28-64; column 6, lines 4-11; column 11, line 66 - column 12, line 16; column 12, lines 23-35 and column 13, lines 1-20 and 51-56. | 13-17 |
| A | KR 10-2001-0105311 A (SCA HYGIENE PRODUCTS ZEIST B.V.) 28 November 2001 (2001-11-28)<br>See claims 1-15. | 1-19 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 December 2023** | **08 December 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2023/013094**

**C. DOCUMENTS CONSIDERED TO BE RELEVANT**

| Category* | Citation of document, with indication, where appropriate, of the relevant passages | Relevant to claim No. |
|---|---|---|
| A | KR 10-2021-0070002 A (HANWHA SOLUTIONS CORPORATION) 14 June 2021 (2021-06-14) See claims 1-13. | 1-19 |
| A | JP 2011-525556 A (CONSTRUCTION RESEARCH & TECHNOLOGY GMBH) 22 September 2011 (2011-09-22) See claims 1-46. | 1-19 |

Form PCT/ISA/210 (second sheet) (July 2022)

**INTERNATIONAL SEARCH REPORT**
**Information on patent family members**

International application No.
**PCT/KR2023/013094**

| Patent document cited in search report | Publication date (day/month/year) | Patent family member(s) | Publication date (day/month/year) |
|---|---|---|---|
| KR 10-2020-0132929 A | 25 November 2020 | BR 112020006831 A2 | 06 October 2020 |
| | | CA 3107604 A1 | 19 September 2019 |
| | | CN 111836653 A | 27 October 2020 |
| | | EP 3765100 A1 | 20 January 2021 |
| | | JP 2021-517931 A | 29 July 2021 |
| | | US 2021-0039068 A1 | 11 February 2021 |
| | | WO 2019-178102 A1 | 19 September 2019 |
| US 5550189 A | 27 August 1996 | CA 2076732 A1 | 18 October 1993 |
| | | CN 1139437 A | 01 January 1997 |
| | | EP 0566118 A1 | 20 October 1993 |
| | | EP 0739357 A1 | 30 October 1996 |
| | | JP 06-025303 A | 01 February 1994 |
| | | JP 06-268091 A | 22 September 1994 |
| | | JP 06-275761 A | 30 September 1994 |
| | | JP 07-231069 A | 29 August 1995 |
| | | JP 09-504331 A | 28 April 1997 |
| | | JP 3221963 B2 | 22 October 2001 |
| | | KR 10-0119464 B1 | 27 October 1997 |
| | | KR 10-0244422 B1 | 01 February 2000 |
| | | KR 10-0331902 B1 | 21 August 2002 |
| | | US 5497032 A | 05 March 1996 |
| | | US 5804468 A | 08 September 1998 |
| | | WO 95-11925 A1 | 04 May 1995 |
| KR 10-2001-0105311 A | 28 November 2001 | AT 483480 T | 15 October 2010 |
| | | AU 1897500 A | 03 July 2000 |
| | | AU 2000-18975 A1 | 03 July 2000 |
| | | AU 767859 B2 | 27 November 2003 |
| | | BR 9916235 A | 04 September 2001 |
| | | CA 2356849 A1 | 22 June 2000 |
| | | DE 69942840 D1 | 18 November 2010 |
| | | EP 1140229 A1 | 10 October 2001 |
| | | ES 2353083 T3 | 25 February 2011 |
| | | JP 2002-532573 A | 02 October 2002 |
| | | MX PA01006098 A | 27 March 2002 |
| | | NZ 512254 A | 28 November 2003 |
| | | PL 348821 A1 | 17 June 2002 |
| | | RU 2227753 C2 | 27 April 2004 |
| | | SK 7982001 A3 | 05 February 2002 |
| | | TN SN99243 A1 | 31 December 2001 |
| | | US 2004-0236016 A1 | 25 November 2004 |
| | | US 6765042 B1 | 20 July 2004 |
| | | WO 00-35504 A1 | 22 June 2000 |
| | | ZA 200104559 A | 04 June 2002 |
| KR 10-2021-0070002 A | 14 June 2021 | CN 114729165 A | 08 July 2022 |
| | | EP 4071212 A1 | 12 October 2022 |
| | | JP 2023-504656 A | 06 February 2023 |
| | | US 2023-0340238 A1 | 26 October 2023 |
| | | WO 2021-112396 A1 | 10 June 2021 |
| JP 2011-525556 A | 22 September 2011 | AU 2009-262378 A1 | 30 December 2009 |
| | | BR PI0913955 A2 | 20 October 2015 |

Form PCT/ISA/210 (patent family annex) (July 2022)

**INTERNATIONAL SEARCH REPORT**
**Information on patent family members**

International application No.
**PCT/KR2023/013094**

| Patent document cited in search report | Publication date (day/month/year) | Patent family member(s) | Publication date (day/month/year) |
|---|---|---|---|
| | | CA 2725995 A1 | 30 December 2009 |
| | | CN 102083874 A | 01 June 2011 |
| | | DE 102008030712 A1 | 31 December 2009 |
| | | EP 2294098 A2 | 16 March 2011 |
| | | MX 2010014522 A | 20 July 2011 |
| | | US 2011-0095227 A1 | 28 April 2011 |
| | | WO 2009-156229 A2 | 30 December 2009 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020220110608


### Non-patent literature cited in the description


- *Potato Research*, 1988, vol. 31, 241-246